# EUROPÄISCHE PATENTSCHRIFT

(11) **EP 2 913 575 B1**
(45) Veröffentlichungstag und Bekanntmachung des Hinweises auf die Patenterteilung: **16.11.2016**
(21) Anmeldenummer: 14157331.1
(22) Anmeldetag: 28.02.2014
(51) Int. Cl.: F16L 33/035

(54) **Schlauchklemme**
Hose clamp
Bride de serrage pour tuyaux

(43) Veröffentlichungstag der Anmeldung: 02.09.2015
(73) Patentinhaber: Oetiker Schweiz AG, 8810 Horgen (CH)
(72) Erfinder: Müller, Manuel, 8966 Oberwil-Lieli (CH)
(74) Vertreter: Strehl Schübel-Hopf & Partner

(56) Entgegenhaltungen:
- DE-U- 7 210 034
- US-A- 3 293 709
- US-A- 4 299 012
- US-A- 4 468 840
- US-A- 4 712 278

## Beschreibung

### Stand der Technik

Aus WO 2013/060346 A ist eine Schlauchklemme aus einem Klemmband bekannt, dessen Enden im geschlossenen Zustand der Klemme unter Bildung eines inneren und eines äußeren Klemmbandabschnitts einander überlappen, wobei einer der Klemmbandabschnitte an seinem Ende in eine Zunge mit gegenüber dem Hauptteil des Klemmbandes verringerter Breite übergeht und die Zunge mindestens einen Vorsprung trägt, der mit mindestens einem am anderen Klemmbandabschnitt vorgesehenen Vorsprung zum Spannen der Klemme zusammenwirkt. Zum Spannen der Klemme um einen zu klemmenden Gegenstand dient dort eine ohrartige Spanneinrichtung. Im gespannten Zustand wird die Klemme durch Eingriff zwischen einer am inneren Klemmbandabschnitt vorgesehenen Außenverzahnung und einer am äußeren Klemmbandabschnitt vorgesehenen Innenverzahnung gehalten. In einer Ausgestaltung ist die Außenverzahnung auf einer am Ende des inneren Klemmbandabschnitts ansetzenden Zunge mit gegenüber dem Hauptteil des Klemmbandes verringerter Breite ausgebildet.

Aus US 4,053,965 ist eine offene Schlauchklemme bekannt, bei der die Endabschnitte des Klemmbandes mit gezahnten Bereichen zum gegenseitigen Eingriff im überlappten Zustand versehen sind. Ein dort nicht näher erläuterter Clip dient dazu, die Endbereiche in Eingriff miteinander zu halten. Ein solcher Clip ist aus US 3,078,532 bekannt. US 1,804,725 zeigt eine weitere Schlauchklemme, bei der ein an der Außenseite einer Zunge vorgesehener erster gezahnter Bereich mit einem an der Innenseite eines erhabenen Bereichs ausgebildeten zweiten gezahnten Bereich zusammenarbeitet. Diese mit Zahneingriff arbeitenden Schlauchklemmen werden bei der Montage durch Ziehen am freien äußeren Ende des Klemmbandes gespannt.

Schlauchklemmen mit den im Oberbegriff des Anspruchs 1 angegebenen Merkmalen sind aus DE 72 100 34 U und US 3,293,709 bekannt. Gegenüber den zuerst genannten Schlauchklemmen mit innen liegender verzahnter Zunge sind beim Spannen dieser Schlauchklemmen geringere Reibungskräfte zu überwinden. Ferner findet der Spannvorgang an der vom Abbindegut abgewandten Seite der Klemme statt und ist deshalb von diesem weitgehend unabhängig. Dadurch wird auch die Gefahr von Beschädigungen des Abbindegutes verringert.

### Abriss der Erfindung

Der Erfindung liegt die Aufgabe zugrunde, Nachteile, wie sie bei vergleichbaren Schlauchklemmen nach dem Stand der Technik auftreten, mindestens teilweise zu beseitigen. Eine speziellere Aufgabe der Erfindung kann darin gesehen werden, eine Schlauchklemme anzugeben bei der ein Ausknicken der Zunge verhindert ist und die auch bei kleinen Durchmessern und größerem Spannweg realisierbar ist.

Die Lösung dieser Aufgabe gelingt mit der in Anspruch 1 definierten Schlauchklemme. Bei dieser trägt eine am äußeren Klemmbandabschnitt vorhandene Zunge an ihrer Innenseite mindestens einen nach innen gerichteten Vorsprung, der mit mindestens einem am inneren Klemmbandabschnitt vorgesehenen nach außen gerichteten Vorsprung zusammenwirkt, und der innere Klemmbandabschnitt weist eine die Zunge mindestens teilweise umgreifende Halterung auf. Die ineinander greifenden, formschlüssig in Eingriff gehaltenen Vorsprünge lassen kein Rückfedern nach dem Spannen zu, so dass ein Verlust an Spannkraft vermieden wird. Am inneren Klemmbandabschnitt sind mehrere an ihrer Außenseite geschlossene Halterungen für die außen liegende Zunge vorgesehen Die aus dem Klemmband ausgestanzten Fenster sind durch Verbreiterung der verbleibenden Materialstege klein gehalten, so dass die Gefahr, dass weiches Schlauchmaterial aus ihnen herausquillt und verletzt wird, gering ist.

Vorzugsweise ist mindestens einer der Vorsprünge als Zahnreihe ausgebildet, so dass sich die Schlauchklemme auf unterschiedliche Durchmesser spannen lässt und Toleranzen im Abbindegut ausgleichen kann.

Sind die Halterungen gemäß einer Ausgestaltung der Schlauchklemme schräg zur Längsrichtung des Klemmbandes verlaufend gestaltet, so ergibt sich eine in Umfangsrichtung ununterbrochene Führung der Zunge.

Vorzugsweise ist am inneren und am äußeren Klemmbandabschnitt jeweils eine Anformung zum Ansetzen eines zangenartigen Spannwerkzeugs ausgebildet, wobei die Anformung am inneren Klemmbandabschnitt nahe dem nach außen gerichteten Vorsprung angeordnet ist und zwei auf beiden Seiten der Zunge angeordnete Haken aufweist. Dadurch wird die Spannkraft in unmittelbarer Nähe der Elemente eingeleitet, die die Schlauchklemme im gespannten Zustand halten.

Zur weiteren Führung der Zunge kann auf der dem inneren Klemmbandende zugewandten Seite der Haken eine weitere Halterung für die Zunge vorgesehen sein.

In weiterer Ausbildung kann der innere Klemmbandabschnitt an seinem Ende in eine innere Zunge mit gegenüber dem Hauptteil des Klemmbandes verringerter Breite übergehen, wobei im äußeren Klemmbandabschnitt eine der Dicke der inneren Zunge entsprechende Stufe mit einem Ausschnitt zum Durchtritt der inneren Zunge ausgebildet ist.

Der an die Stufe anschließende Bereich des Klemmbandes kann ein der Breite der inneren Zunge entsprechendes Fenster aufweisen, das durch den verbleibenden Materialstreifen des Klemmbandes abgedeckt ist. Diese Maßnahmen gewährleisten eine unterbrechungslose Unterstützung des Abbindegutes durch die Schlauchklemme.

Vorzugsweise wird die Schlauchklemme im geschlossenen, ungespannten Zustand geliefert. Zur Transportsicherung kann die innere Zunge an ihrem Ende einen Haken zum Einhängen an der Stufe im ungespannten Zustand der Klemme aufweisen.

### Kurzbeschreibung der Zeichnungen

Ausführungsbeispiele der Erfindung werden nachstehend anhand der Zeichnungen näher erläutert. Darin zeigen:
Fig. 1 eine perspektivische Darstellung einer Schlauchklemme im gestreckten Zustand;
Fig. 2 einen Axialschnitt durch die Schlauchklemme nach Fig. 1 im geschlossenen, nicht gespannten Zustand;
Fig. 3 einen Axialschnitt durch die Schlauchklemme nach Fig. 1 und 2 im geschlossenen und gespannten Zustand;
Fig. 4 und 5 perspektivische Darstellungen der Schlauchklemme nach Fig. 1 bis 3 im geschlossenen, gespannten Zustand;
Fig. 6 bis 8 jeweils einen Teil der Schlauchklemme im gestreckten Zustand in unterschiedlichen Gestaltungen.

### Ausführungsbeispiele

In der folgenden Beschreibung beziehen sich die Ausdrücke "innen" und "außen" auf die Seiten der Schlauchklemme, die im geschlossenen Zustand dem zu klemmenden Schlauch (Abbindegut) zugewandt bzw. von ihm abgewandt sind.

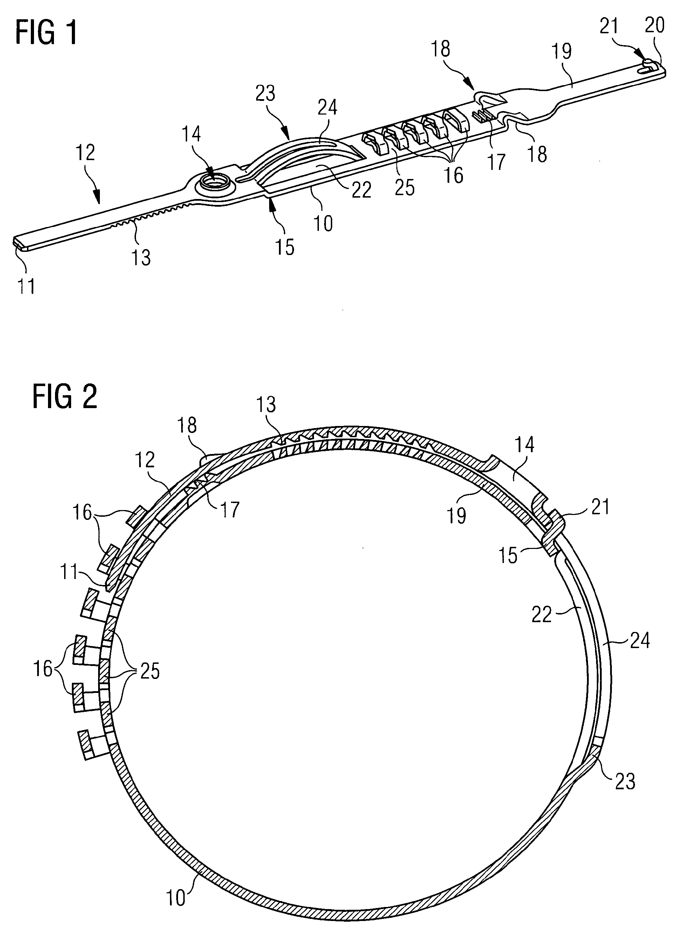

Die in Fig. 1 bis 5 gezeigte Schlauchklemme besteht aus einem Klemmband **10,** das, ausgehend von dem in Fig. 1 linken, äußeren Ende **11,** folgende Elemente aufweist: eine äußere Zunge **12,** deren Breite geringer ist als die volle Breite des Klemmbandes **10** und die an ihrer inneren, in Fig. 1 unteren Seite einen ersten gezahnten Bereich **13** trägt, eine am Beginn des Bereiches voller Klemmbandbreite aus dem Klemmbandmaterial herausgeprägte, nach außen offene Öse **14,** eine nach innen springende Stufe **15,** deren Höhe der Dicke des Klemmbandmaterials entspricht, mehrere, in dem gezeigten Beispiel sechs, aus dem Klemmbandmaterial herausgeprägte Führungsbügel **16,** einen nach außen weisenden zweiten gezahnten Bereich **17,** zwei seitliche Haltehaken **18** und innere Zunge **19,** deren Breite ebenfalls geringer ist als die volle Klemmbandbreite und die nahe ihrem Ende **20** einen nach außen ragenden und in Richtung des linken Endes **11** des Klemmbandes **10** offenen Sicherungshaken **21** trägt.

In dem in Fig. 2 gezeigten geschlossenen, nicht gespannten Zustand, in dem die Schlauchklemme vom Hersteller ausgeliefert wird, liegt die innere Zunge **19** in einem aus dem Klemmband **10** teilweise ausgestanzten Fenster **22,** das an der Stufe **15** beginnt. Das aus dem Fenster **22** ausgeschnittene Klemmbandmaterial ist entsprechend der Krümmung der geschlossenen Klemme vorgebogen und bildet eine Abdeckung **23,** die an ihren Ende mit dem Klemmband voller Breite zusammenhängt und die innere Zunge **19** an einem Ausweichen nach außen hindert. In dem in Fig. 2 gezeigten geschlossenen, nicht gespannten Zustand ragt der Sicherungshaken **21** durch einen in der Abdeckung **23** ausgebildeten, in Umfangsrichtung verlaufenden Schlitz **24** hindurch und greift am äußeren Klemmbandabschnitt nahe der Öse **14** an.

Wie in Fig. 2 gezeigt, verläuft die äußere Zunge **12** zwischen den seitlichen Haltehaken **18,** überquert den zweiten gezahnten Bereich **17** und durchsetzt im nicht gespannten Zustand mindestens den vom äußeren Klemmbandende **11** am weitesten entfernten Führungsbügel **16** und, wie in Fig. 2 dargestellt, auch den folgenden Führungsbügel. Dadurch wird die äußere Zunge **12** eng an dem inneren Klemmbandteil gehalten.

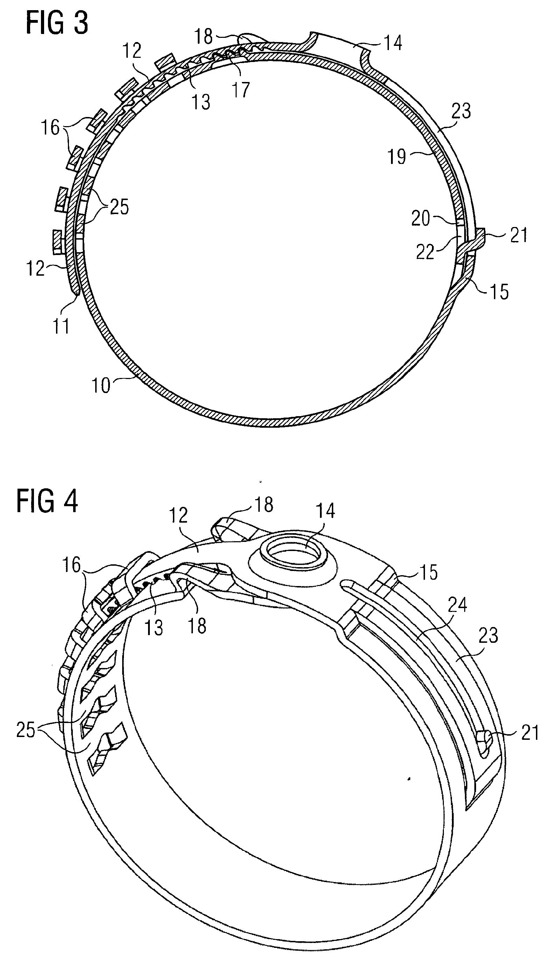

Nach Anbringen auf das (nicht dargestellte) Abbindegut, typisch einen auf einen Rohrnippel aufgeschobenen Schlauch, wird die Schlauchklemme mittels eines an der Öse **14** und den Haltehaken **18** zangenartig angreifenden Spannwerkzeugs verengt und um das Abbindegut gespannt, wobei sich die äußere Zunge **12,** wie aus Fig. 3 bis 5 ersichtlich, unter Führung durch die Führungsbügel **16** weiter über den inneren Klemmbandteil schiebt. Dabei gelangt der erste gezahnte Bereich **13** der äußeren Zunge **12** in Eingriff mit dem zwischen dem Haltehaken **18** und den Führungsbügeln **16** liegenden zweiten gezahnten Bereich **17.** In diesem Eingriff wird die äußere Zunge **12** durch die Führungsbügel **16** gehalten.

Bei diesem Spannvorgang der Schlauchklemme löst sich der Sicherungshaken **21** aus seinem Eingriff mit dem äußeren Klemmbandteil und verschiebt sich längs des Schlitzes **24.** Dabei bewirkt der Sicherungshaken **21** eine Führung der inneren Zunge **19** in Umfangsrichtung der Schlauchklemme.

Die Führungsbügel **16** sind aus dem Klemmband herausgeprägt. Die zwischen den Führungsbügeln **16** verbleibenden Stege **25** werden seitlich verprägt, um die Lücken im Klemmband zu verkleinern und die Auflagefläche für das Abbindegut zu vergrößern und dessen Herausquellen zu verringern. Dadurch wird auch die Gefahr von Beschädigungen des Abbindegutes vermindert.

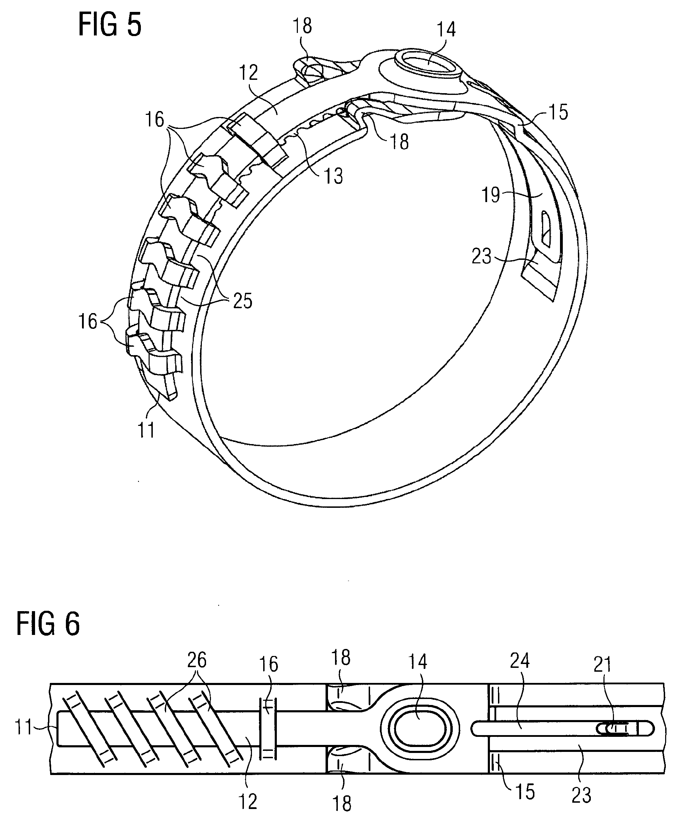

Die insbesondere in Fig. 1 und 5 sichtbare geschweifte Gestaltung der Kanten der Führungsbügel **16** und der Stege **25** verbessert die Führung der äußeren Zunge **12.**

In den in Fig. 6 bis 8 gezeigten Varianten verlaufen die Führungsbügel **26** schräg zur Achse der Schlauchklemme. Dadurch wird die äußere Zunge **12** in Umfangsrichtung praktisch ununterbrochen geführt.

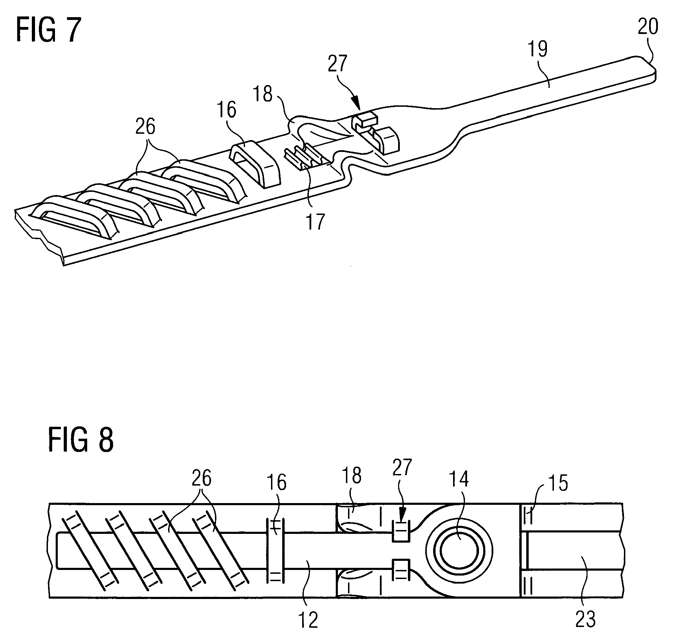

Die Gestaltung nach Fig. 7 und 8 unterscheidet sich von der nach Fig. 1 bis 5 darin, dass auf der vom äußeren Klemmbandende **11** abgewandten Seite der seitlichen Haltehaken **18** ein radial offener Haltebügel **27** vorgesehen ist, der den Eingriff zwischen den verzahnten Bereichen **13** und **17** und die Sicherheit gegen äußere Einflüsse verbessert. Ferner übernimmt der Haltehaken die Funktion eines Anschlags bei maximaler Spannung der Schlauchklemme. Zusätzlich vereinfacht er den Rundungsprozess in der Fertigung.

Die oben beschriebene Schlauchklemme zeichnet sich durch folgende Eigenschaften aus:
a) Geringer Kraftverlust beim Spannen, da anders als bei sogenannten Ohrklemmen keine plastische Verformung erfolgt und im Gegensatz zu einer inneren verzahnten Zunge geringere Reibungskräfte zu überwinden sind.
b) Toleranzausgleich durch verschiedene Einhängepositionen zwischen den verzahnten Bereichen **13, 17,** wodurch auch unterschiedliche Durchmesser der gespannten Schlauchklemme möglich werden.
c) Geringe Knickgefahr der äußeren, verzahnten Zunge **12** durch die widerstandsarme Zungenführung mittels der Führungsbügel **16,** wodurch auch bei kleinen Durchmessern das verzahnte Funktionsprinzip mit großem Spannweg realisierbar ist.
d) Niedrige Bauhöhe durch das Element "Öse" **14,** das den Angriff der Spannkraft im Wesentlichen in der Ebene des Klemmbandes ermöglicht. Werden - in einer Variante - die seitlichen Haltehaken **18** durch eine zweite Öse ähnlich der Öse **14** ersetzt, so ergibt sich der Vorteil, dass das Spannwerkzeug relativ zur Schlauchklemme leichter auszurichten ist.
e) Da direkt zum Eingriff der verzahnten Bereiche **13, 17** unter diesen keine Reaktionskräfte auftreten, ist der Spannvorgang ist im Wesentlichen unabhängig von den Eigenschaften des Abbindegutes.
f) Am Abbindegut auftretende Expansionskräfte führen zu einer Verbesserung der Verriegelung zwischen den verzahnten Bereichen **13, 17.**
g) Die Stufe **15** in Verbindung mit der Abdeckung **22** ergibt eine gleichmäßige und lückenlose Anpressung der Schlauchklemme über den gesamten Umfang des Abbindegutes.

### Bezugszeichen

- **10**: Klemmband
- **11**: äußeres Klemmbandende
- **12**: äußere Zunge
- **13**: erster gezahnter Bereich
- **14**: Öse
- **15**: Stufe
- **16**: Führungsbügel
- **17**: zweiter gezahnter Bereich
- **18**: Haltehaken
- **19**: innere Zunge
- **20**: inneres Klemmbandende
- **21**: Sicherungshaken
- **22**: Fenster
- **23**: Abdeckung
- **24**: Schlitz
- **25**: Stege
- **26**: Führungsbügel
- **27**: Haltebügel

## Patentansprüche

1. Schlauchklemme aus einem Klemmband **(10),** dessen Enden im geschlossenen Zustand der Klemme unter Bildung eines inneren und eines äußeren Klemmbandabschnitts einander überlappen, wobei der äußere Klemmbandabschnitt an seinem Ende in eine Zunge **(12)** mit gegenüber dem Hauptteil des Klemmbandes verringerter Breite übergeht und die Zunge **(12)** an ihrer Innenseite mindestens einen nach innen gerichteten Vorsprung **(13)** trägt, der mit mindestens einem am inneren Klemmbandabschnitt vorgesehenen, nach außen gerichteten Vorsprung **(17)** zum Spannen der Klemme zusammenwirkt, und wobei der innere Klemmbandabschnitt mehrere die Zunge **(12)** mindestens teilweise umgreifende, an ihrer Außenseite geschlossene Halterungen **(16; 26)** aufweist
**dadurch gekennzeichnet, dass** die Halterungen **(16; 26)** aus dem Material des Klemmbandes herausgeprägt und die zwischen benachbarten Halterungen verbleibenden Materialstege **(25)** verbreitert sind.

2. Schlauchklemme nach Anspruch 1, wobei die Zunge **(12)** mehrere nach innen gerichtete Zähne **(13)** trägt.

3. Schlauchklemme nach Anspruch 1, wobei die Halterungen **(26)** schräg zur Längsrichtung des Klemmbandes **(10)** verlaufen.

4. Schlauchklemme nach einem der vorhergehenden Ansprüche, wobei am inneren und am äußeren Klemmbandabschnitt jeweils eine Anformung **(14, 18)** zum Ansetzen eines zangenartigen Spannwerkzeugs ausgebildet und die Anformung **(18)** am inneren Klemmbandabschnitt nahe dem nach außen gerichteten Vorsprung **(17)** angeordnet ist.

5. Schlauchklemme nach Anspruch 4, wobei die am inneren Klemmbandabschnitt vorgesehene Anformung zwei auf beiden Seiten der Zunge **(12)** angeordnete Haken **(18)** aufweist.

6. Schlauchklemme nach Anspruch 5, wobei auf der dem inneren Klemmbandende zugewandten Seite der Haken **(18)** eine weitere Halterung für die Zunge vorgesehen ist.

7. Schlauchklemme nach einem der vorhergehenden Ansprüche, wobei der innere Klemmbandabschnitt an seinem Ende in eine innere Zunge **(19)** mit gegenüber dem Hauptteil des Klemmbandes **(10)** verringerter Breite übergeht und im äußeren Klemmbandabschnitt eine der Dicke der inneren Zunge **(19)** entsprechende Stufe **(15)** ausgebildet ist.

8. Schlauchklemme nach Anspruch 7, wobei der an die Stufe **(15)** anschließende Bereich des Klemmbandes **(10)** ein der Breite der inneren Zunge entsprechendes Fenster **(22)** aufweist.

9. Schlauchklemme nach Aspruch 8, wobei das Fenster **(22)** durch den verbleibenden Materialstreifen **(23)** des Klemmbandes **(10)** abgedeckt ist, der längs einem Bogens mit einer der gespannten Schlauchklemme entsprechenden Krümmung verläuft.

## Claims

1. A hose clamp made of a clamping band **(10)** the ends of which overlap one another in the closed condition of the clamp thereby forming an inner and an outer clamping band portion, wherein the outer clamping band portion has its end terminating in a tongue **(12)** which has a width reduced with respect to that of the main portion of the clamping band, and the tongue **(12)** carries on its inner side at least one inward facing projection **(13)** cooperating with at least one outward facing projection provided at the inner clamping band portion for tightening the clamp, and wherein the inner clamping band portion has a plurality of retainers (16; 26) which are closed at their outer sides and surround the tongue **(12)** at least in part,
**characterised in that** the retainers **(16; 26)** are stamped from the material of the clamping band, and the webs **(25)** left between adjacent retainers are enlarged in width.

2. The hose clamp of claim 1, wherein the tongue **(12)** carries a plurality of inward facing teeth **(13).**

3. The hose clamp of claim 1, wherein the retainers **(26)** extend at an angle to the longitudinal direction of the clamping band **(10).**

4. The hose clamp of any preceding claim, wherein each of the inner and outer clamping band portions has a formation **(14, 18)** for engagement by a plier-type tightening tool, and the formation **(18)** on the inner clamping band portion is disposed close to the outward facing projection **(17).**

5. The hose clamp of claim 4, wherein the formation provided on the inner clamping band portion has a pair of hooks (**18**) disposed on either side of the tongue **(12).**

6. The hose clamp of claim 5, wherein a further retaining structure for the tongue is provided on the side of the hooks **(18)** facing the inner clamping band end.

7. The hose clamp of any preceding claim, wherein the end of the inner clamping band portion terminates in an inner tongue **(19)** of a width reduced with respect to the main portion of the clamping band (**10**) and a step **(15)** corresponding to the thickness of the inner tongue **(19)** is formed in the outer clamping band portion.

8. The hose clamp of claim 7, wherein the region of the clamping band **(10)** adjacent to the step **(15)** has a window **(22)** corresponding to the width of the inner tongue.

9. The hose clamp of claim 8, wherein the window **(22)** is covered by the remaining material strip **(23)** of the clamping band **(10)** which extends along an arc having a curvature corresponding to that of the tightened hose clamp.

## Revendications

1. Collier de serrage pour tuyau constitué d'une bande de serrage (10), dont les extrémités se chevauchent lorsque le collier est serré en formant une portion de bande de serrage intérieure et une portion de bande de serrage extérieure, dans lequel la portion de bande de serrage extérieure se transforme à son extrémité en une languette (12) avec une largeur réduite par rapport à la partie principale de la bande de serrage, et la languette (12) supporte sur son côté intérieur au moins une saillie (13) dirigée vers l'intérieur, laquelle saillie coopère avec au moins une saillie (17) dirigée vers l'extérieur, prévue sur la portion de bande de serrage intérieure pour serrer le collier, et dans lequel la portion de bande de serrage intérieure comporte plusieurs éléments de retenue (16 ; 26) fermés sur leur côté extérieur et s'engageant au moins partiellement autour de la languette (12),
**caractérisé en ce que** les éléments de retenue (16 ; 26) sont estampés vers l'extérieur à partir du matériau de la bande de serrage et les branches de matériau (25) restant entre des éléments de retenue voisins sont élargies.

2. Collier de serrage pour tuyau selon la revendication 1, dans lequel la languette (12) supporte plusieurs dents (13) dirigées vers l'intérieur.

3. Collier de serrage pour tuyau selon la revendication 1, dans lequel les éléments de retenue (26) s'étendent en biais par rapport à la direction longitudinale de la bande de serrage (10).

4. Collier de serrage pour tuyau selon l'une des revendications précédentes, dans lequel sur la portion de bande de serrage intérieure et sur la portion de bande de serrage extérieure est respectivement formé un appendice (14, 18) destiné à appliquer un outil de serrage analogue à une pince et l'appendice (18) est agencé sur la portion de bande de serrage intérieure près de la saillie (17) dirigée vers l'extérieur.

5. Collier de serrage pour tuyau selon la revendication 4, dans lequel l'appendice prévu sur la portion de bande de serrage intérieure comporte deux crochets (18) agencés des deux côtés de la languette (12).

6. Collier de serrage pour tuyau selon la revendication 5, dans lequel sur le côté des crochets (18) dirigé vers l'extrémité de bande de serrage intérieure est prévu un élément de retenue supplémentaire pour la languette.

7. Collier de serrage pour tuyau selon l'une des revendications précédentes, dans lequel la portion de bande de serrage intérieure se transforme à son extrémité en une languette intérieure (19) avec une largeur réduite par rapport à la partie principale de la bande de serrage (10), et un gradin (15) correspondant à l'épaisseur de la languette intérieure (19) est formé dans la portion de bande de serrage extérieure.

8. Collier de serrage pour tuyau selon la revendication 7, dans lequel la zone de la bande de serrages(10) adjacente au gradin (15) comporte une fenêtre (22) correspondant à la largeur de la languette intérieure.

9. Collier de serrage pour tuyau selon la revendication 8, dans lequel la fenêtre (22) est recouverte par la bande de matière restante (23) de la bande de serrage (10), laquelle bande s'étend le long d'un arc ayant une courbure correspondant au collier de serrage pour tuyau serré.
